# EUROPEAN PATENT APPLICATION

(11) **EP 4 559 457 A1**
(43) Date of publication of application: **28.05.2025**
(21) Application number: 24214088.7
(22) Date of filing: 20.11.2024
(51) Int. Cl.: A61K 9/20, A61K 9/28

(54) **A MODIFIED RELEASE TABLET COMPOSITION OF BRIVARACETAM**

(30) Priority: 22.11.2023 TR 202315500
(71) Applicant: Sanovel Ilac Sanayi ve Ticaret A.S., Sisli - Istanbul (TR)
(72) Inventor: SUNEL, Fatih, Istanbul (TR); BILGEHAN ATAK, Fadime, Istanbul (TR); ATAMAN, Seval, Istanbul (TR); DERELI, Selin, Istanbul (TR)
(74) Representative: Genç Ilhan, Oznur

(57) **Abstract**

The present invention relates to a modified release tablet composition comprises brivaracetam and at least one diluent wherein the amount of diluents is 32.0% to 55.0% by weight in the total composition and further comprising a release rate-controlling membrane coating. Furthermore, the tablet is also obtained by using an effective process which is simple, rapid, cost effective, time-saving and industrially convenient process.

## Description

### Field of the Invention

The present invention relates to a modified release tablet composition comprises brivaracetam and at least one diluent wherein the amount of diluents is 32.0% to 55.0% by weight in the total composition and further comprising a release rate-controlling membrane coating. Furthermore, the tablet is also obtained by using an effective process which is simple, rapid, cost effective, time-saving and industrially convenient process.

### Background of the Invention

The chemical name of brivaracetam is (2S)-2-[(4R)-2-oxo-4-propyl-pyrrolidin-1-yl]butanamide and its chemical structure is shown in the Formula I.

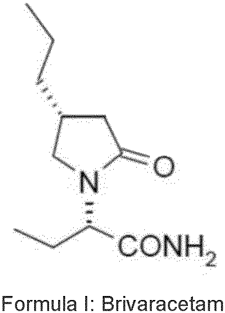

The European Medicines Agency (EMA) and the US Food and Drug Administration (FDA) approved brivaracetam as an adjunctive therapy drug for the treatment of partial seizures with or without secondary generalized seizures of epilepsy patients of 16-year-old and older, under the trade name Briviact^{®}. The existing commercially-available preparations comprise a brivaracetam tablet, a brivaracetam oral liquid and a brivaracetam injection, wherein the brivaracetam tablet has a plurality of specifications such as 10 mg, 25 mg, 50 mg, 75 mg and 100 mg, and both the brivaracetam oral liquid and the brivaracetam injection have a specification of 10 mg/mL.

Brivaracetam is highly soluble and is Class I according to the Biopharmaceutical Classification System (BCS). The active substance is a white to off-white non-hygroscopic crystalline solid. Additionally, brivaracetam is an extremely viscous compound (adhesive capacity). Brivaracetam has short half-life and high-water solubility. These characteristics are ideal to develop modified release pharmaceutical compositions which invariably will offer various advantages over conventional immediate release drug composition such as constant therapeutic plasma concentration of Brivaracetam over prolonged periods, reduced number of doses per day or week, reduced adverse effects and improved patient compliance and convenience.

A modified release formulation would be particularly desirable for administration in some patients. A prolonged release formulation could be advantageously used in order to reduce the difference between plasmatic Cₘₐₓ and Cₘᵢₙ and consequently to lower sides effects. Moreover, a prolonged release formulation improves the patient's compliance as the administration frequency could be reduced.

Patent WO 2009/144286 discloses a pharmaceutical composition in the form of a film coated tablet comprising Brivaracetam and, as excipient within the core of the tablet, 5% to 80% per weight of at least one hydrophilic matrix agent for prolonged release.

US2013039957 discloses a controlled release pharmaceutical compositions comprising Brivaracetam or its pharmaceutically acceptable derivatives thereof.

There still remains a need in the art to provide an improved a modified release tablet comprising brivaracetam. In the present invention, the modified release tablet is created to overcome the above problems and provides additional advantages to the relevant field of art. Other advantages and embodiments of the present invention will be clarified in the following description.

### Detailed Description of the Invention

The main object of the present invention is to control the release of brivaracetam and ensure a long-term effective therapeutic effect when administered to the patient which overcomes the above-described problems in the prior art and have additive advantages over them.

Another object of the present invention is to provide a modified release tablet comprising brivaracetam with excellent properties, such as compressibility, flowability, homogeneity, content uniformity and stability and the desired dissolution profile.

Another object of the present invention is to provide a process for preparing a modified release tablet comprising brivaracetam. The process is free of solvent, for example dry granulation or direct compression or hot melt extrusion. It is a simple, rapid, cost effective, time-saving, and industrially convenient method.

The term "modified release" refers to any pharmaceutical formulation that maintain constant levels of a drug in the patient's bloodstream by releasing the drug over an extended period of time. Modified release is formulated to release the active ingredient gradually and predictably over a 12-hour to 24-hour period. Modified release is selected from the group comprising controlled release, sustained release, delayed release, extended release, repeat action system or mixtures thereof.

Content uniformity is an important parameter in tablet formulation. Especially the high sticky behavior of the brivaracetam caused content uniformity problems in formulations. The problem with flowability negatively affects the dissolution profile. So, we created a composition that ensures content uniformity and also provides the desired delayed release profile. We have found that these problems are overcome when using diluents at the specified ranges. We also used a release rate-controlling membrane coating for the desired release profile in a way that would not have a negative impact on the composition.

According to one embodiment of this invention, a modified release tablet composition comprises brivaracetam and at least one diluent wherein the amount of diluents is 32.0% to 55.0% by weight in the total composition and further comprising a release rate-controlling membrane coating.

Suitable diluents are selected from the group comprising lactose, lactose monohydrate, lactose anhydrous, microcrystalline cellulose, corn starch, starch, mannitol, calcium hydrogen phosphate dihydrate, dicalcium hydrogen phosphate anhydrate, calcium phosphate trihydrate, neutral pellets, magnesium carbonate, magnesium oxide, maltodextrin, maltose, medium chain triglycerides or mixtures thereof.

According to an embodiment of the present invention, diluent is lactose monohydrate or lactose anhydrous or mixtures thereof. The diluent provides improving the flowability, enhancing the compressive strength or hardness, reducing the friability during the component mixing and tableting.

According to an embodiment of the present invention, the amount of diluents is 32.0% to 50.0% by weight in the total composition. Preferably, the amount of diluents is 34.0% to 47.0% by weight in the total composition.

According to an embodiment of the present invention, the tablet further comprises at least one pharmaceutically acceptable excipient which is selected from the group comprising binders, lubricants or mixtures thereof.

Suitable binders are selected from the group comprising polyvinylpyrrolidone, hydroxypropyl cellulose, sodium carboxymethyl cellulose, polyethylene glycol, starch, pregelatinized starch, sodium alginate, hydroxypropyl methyl cellulose, carboxy methyl cellulose, methyl cellulose, guar gum, polymethacrylates, methacrylate polymers, polysaccharides, poloxamer, polyoxyethylene-alkyl ether, polydextrose, polyethylene oxide or mixtures thereof.

According to an embodiment of the present invention, the binder is polyvinylpyrrolidone.

According to an embodiment of the present invention, the amount of binders is 20.0% to 40.0% by weight in the total composition. Preferably, the amount of binders is 27.0% to 37.0% by weight in the total composition.

Suitable lubricant is selected from the group comprising talc, sodium stearyl fumarate, magnesium stearate, potassium stearate, stearic acid, sodium chloride, sodium benzoate, sodium acetate, sodium oleate, polyethylene glycols or mixtures thereof.

According to one embodiment of the present invention, the lubricant is talc or magnesium stearate or mixtures thereof.

According to one embodiment of the present invention, the amount of lubricant is between 0.2% and 5.0% by weight in the total composition.

According to one embodiment of the present invention, the amount of release rate-controlling membrane coating is between 3.0% and 15.0% by weight in the total composition. This helps to affect positively the desired dissolution profile.

According to one embodiment of this invention, the release rate-controlling membrane coating comprises at least one polymer and at least one plasticizer.

Suitable a polymer is selected from the group comprising ethyl cellulose, hydroxypropyl methylcellulose, polymethacrylate, hydroxy propyl cellulose, polyvidone or mixtures thereof.

According to one embodiment of this invention, the polymer is ethyl cellulose and hydroxypropyl methylcellulose (HPMC).

According to an embodiment of the present invention, the amount of ethyl cellulose is 30.0% to 70.0% by weight in the total coating.

According to an embodiment of the present invention, the amount of HPMC is 15.0% to 40.0% by weight in the total coating.

The addition of the polymer is especially useful to increase the onset of action. For the particles according to the present invention, the ratio ethylcellulose:HPMC is vary from 1:1 to about 4:1, in particular from about 1.5:1 to about 3:1.

Suitable plasticizer is selected from the group comprising dibutyl sebacate, dimethyl phthalate, diethyl phthalate, triethyl citrate, tributyl citrate, chlorobutanol, dibutyl phthalate or mixtures thereof.

According to one embodiment of this invention, the plasticizer is dibutyl sebacate and triethyl citrate.

According to an embodiment of the present invention, the amount of dibutyl sebacate is 3.0% to 25.0% by weight in the total coating.

According to an embodiment of the present invention, the amount of triethyl citrate is 0.5% to 15.0% by weight in the total coating.

We found that there is no flowability problem when the active ingredient is used in the particle sizes specified below.

As used here in, 'particle size' means the cumulative volume size distribution as tested by any conventionally accepted method such as the laser diffraction method (i.e. Malvern analysis). The term d (0.1) means, the size at which 10% by volume of the particles are finer and d (0.5) means the size at which 50% by volume of the particles are finer and d (0.9) means the size at which %90 by volume of the particles is finer.

According to one embodiment of this invention, brivaracetam has a d (0.9) particle size 40 µm to 480 µm.

According to one embodiment of this invention, brivaracetam has a d (0.9) particle size between 55 µm to 94 µm or between 75 µm to 96 µm or between 104 µm to 125 µm or between 126 µm to 138 µm or between 139 µm to 152 µm or between 153 µm to 167 µm or between 168 µm to 183 µm or between 184 µm to 198 µm or between 203 µm to 218 µm or between 219 µm to 234 µm or between 237 µm to 253 µm or between 257 µm to 268 µm or between 269 µm to 278 µm or between 283 µm to 298 µm or between 304 µm to 345 µm or between 348 µm to 390 µm or between 395 µm to 445 µm Brivaracetam having above-described particle size provides the flowability and compressibility, mass uniformity also provides the improved dissolution profile.

According to one embodiment of this invention, brivaracetam has a d (0.1) particle size between 1 µm to 40 µm or between 4 µm to 34 µm or between 6 µm to 25 µm.

According to one embodiment of this invention, brivaracetam has a d (0.5) particle size between 8 µm to 85 µm or between 13 µm to 76 µm or between 22 µm to 65 µm or between 27 µm to 58 µm.

According to one embodiment of the present invention, the modified release tablet composition comprises;
- Brivaracetam
- Lactose monohydrate
- Lactose anhydrous
- PVP
- Talc
- Mg stearate

According to one embodiment of the present invention, the release rate-controlling membrane coating comprises;
- Ethyl cellulose
- Dibutyl sebacate
- Triethyl citrate
- HPMC

We found that the brivaracetam is extremely prone to sticking and picking during the preparation process, which brings great problems to the appearance and content uniformity of the final product. We have seen that these problems are overcome when no solvent is used during the process.

In the present invention, the tablet ingredients are not exposed to moisture, solvents. Thus, in this invention, direct compression or dry granulation or hot melt extrusion is used to process moisture, solvent for sensitive brivaracetam. In addition, these methods provide the desired compressibility and flowability without loss of active substance.

According to an embodiment of the present invention, the modified release tablet comprising brivaracetam is obtained by direct compression or dry granulation.

### Example 1: The tablet comprising release rate-controlling membrane coating

| **Ingredients** | **% by weight** | **% by weight** |
|---|---|---|
| Brivaracetam | 5.0 - 22.0 | 16.8 |
| Lactose monohydrate | 1.0 - 15.0 | 5.4 |
| Lactose anhydrous | 20.0 - 40.0 | 34.0 |
| PVP | 20.0 - 40.0 | 34.0 |
| Talc | 0.1 - 3.0 | 0.7 |
| Mg stearate | 0.1 - 3.0 | 0.7 |
| Release Rate-Controlling membrane coating | 3.0 - 15.0 | 9.0 |
| **Total** | **100** | **100** |

A process for example 1;
a) Mixing Brivaracetam, Lactose monohydrate, Lactose anhydrous, PVP for 15 minutes,
b) Adding Talc and mg stearate to dry mixture and mixing for 3 minutes,
c) Compressing the mixture into a core tablet following appropriate specifications,
d) Coating the core tablet with Release Rate-Controlling membrane coating.

### Example 2: Release Rate-Controlling membrane coating

| **Ingredients** | **% by weight** | **% by weight** |
|---|---|---|
| Ethyl cellulose | 30.0 - 70.0 | 55.0 |
| Dibutyl sebacate | 3.0 - 25.0 | 11.1 |
| Triethyl citrate | 0.5 - 15.0 | 7.4 |
| HPMC | 15.0 - 40.0 | 22.2 |
| Purified water* | q.s | q.s |
| **Total** | **100** | **100** |

| | | |
|---|---|---|
| *: sufficient quantity | | |

## Claims

1. A modified release tablet composition comprises brivaracetam and at least one diluent wherein the amount of diluents is 32.0% to 55.0% by weight in the total composition and further comprising a release rate-controlling membrane coating.

2. The modified release tablet composition according to claim 1, wherein diluents are selected from the group comprising lactose, lactose monohydrate, lactose anhydrous, microcrystalline cellulose, corn starch, starch, mannitol, calcium hydrogen phosphate dihydrate, dicalcium hydrogen phosphate anhydrate, calcium phosphate trihydrate, neutral pellets, magnesium carbonate, magnesium oxide, maltodextrin, maltose, medium chain triglycerides or mixtures thereof.

3. The modified release tablet composition according to claim 2, wherein diluent is lactose monohydrate or lactose anhydrous or mixtures thereof.

4. The modified release tablet composition according to claim 2 or 3, wherein the amount of diluents is 32.0% to 50.0% by weight in the total composition.

5. The modified release tablet composition according to claim 1, wherein further comprises at least one pharmaceutically acceptable excipient which is selected from the group comprising binders, lubricants or mixtures thereof.

6. The modified release tablet composition according to claim 5, wherein binders are selected from the group comprising polyvinylpyrrolidone, hydroxypropyl cellulose, sodium carboxymethyl cellulose, polyethylene glycol, starch, pregelatinized starch, sodium alginate, hydroxypropyl methyl cellulose, carboxy methyl cellulose, methyl cellulose, guar gum, polymethacrylates, methacrylate polymers, polysaccharides, poloxamer, polyoxyethylene-alkyl ether, polydextrose, polyethylene oxide or mixtures thereof.

7. The modified release tablet composition according to claim 6, wherein the binder is polyvinylpyrrolidone.

8. The modified release tablet composition according to claim 6 or 7, wherein the amount of binders is 20.0% to 40.0% by weight in the total composition.

9. The modified release tablet composition according to claim 1, wherein the amount of release rate-controlling membrane coating is between 3.0% and 15.0% by weight in the total composition.

10. The modified release tablet composition according to claim 9, wherein the release rate-controlling membrane coating comprises at least one polymer and at least one plasticizer.

11. The modified release tablet composition according to claim 10, wherein a polymer is selected from the group comprising ethyl cellulose, hydroxypropyl methylcellulose, polymethacrylate, hydroxy propyl cellulose, polyvidone or mixtures thereof.

12. The modified release tablet composition according to claim 10, wherein plasticizer is selected from the group comprising dibutyl sebacate, dimethyl phthalate, diethyl phthalate, triethyl citrate, tributyl citrate, chlorobutanol, dibutyl phthalate or mixtures thereof.

13. The modified release tablet composition according to claim 1, wherein brivaracetam has a d (0.9) particle size 40 µm to 480 µm.

14. The modified release tablet composition according to claim 1, wherein the tablet composition comprises;
• Brivaracetam
• Lactose monohydrate
• Lactose anhydrous
• PVP
• Talc
• Mg stearate

15. The modified release tablet composition according to claim 1, wherein the modified release tablet comprising brivaracetam is obtained by direct compression or dry granulation.
